# EUROPEAN PATENT APPLICATION

(11) **EP 3 354 722 A1**
(43) Date of publication of application: **01.08.2018**
(21) Application number: 16834658.3
(22) Date of filing: 10.08.2016
(51) Int. Cl.: C12N 5/0735

(54) **METHOD FOR CONTROLLING DIFFERENTIATION OF STEM CELLS**

(30) Priority: 11.08.2015 CN 201510491300
(71) Applicant: Guangzhou Institutes of Biomedicine and Health, Chinese Academy of Sciences, Guangdong 510530 (CN)
(72) Inventor: PEI, Duanqing, Guangzhou Guangdong 510530 (CN); SHU, Xiaodong, Guangzhou Guangdong 510530 (CN); LI, Qiuhong, Guangzhou Guangdong 510530 (CN); HUTCHINS, Andrew, Guangzhou Guangdong 510530 (CN)
(74) Representative: J A Kemp
(86) International application number: PCT/CN2016/094380
(87) International publication number: WO 2017/025034

(57) **Abstract**

Provided is a method for controlling differentiation of pluripotent stem cells into endoderm-derived cells, and particularly for preventing, by using a TGF inhibitor or an SNAI1 inhibitor, differentiation of pluripotent stem cells into the endoderm-derived cells.

## Description

### FIELD OF THE INVENTION

The present invention provides methods to control the differentiation of pluripotent stem cells. In particular, the present invention provides methods for controlling the differentiation of pluripotent stem cell toward endoderm originated cells.

### BACKGROUND OF THE INVENTION

Stem cells, having the ability to differentiate into many different cell types that make up an organism, are able to differentiate into derivatives of all three embryonic germ layers (endoderm, mesoderm and ectoderm).

A considerable amount of interest has been generated in stem cell committed differentiation. The fist intermediate stage of differentiation is the formation of definitive layer type cells. For the purpose of obtaining pure targeted cells, it is advantageous that undesired types of cells are prohibited from differentiation. However, very limited study results have been revealed for the inhibition of differentiation toward definite type of cells.

### SUMMARY OF THE INVENTION

The present invention is based on the discovery that endogenous TGFβ activated by Activin A drives epithelila-to-mesenchymal transition (EMT) and definitive endoderm (DE) formation in a SNAI1 dependent fashion. Blocking of SNAI1 or the introducing of TGFβ inhibitor will block the EMT of the stem cells, thereby inhibit the stem cells from the acquisition of endoderm character during differentiation. Therefore, it is controllable that no DE originated cell will be formed. As the DE layer type cells are prohibited at the beginning of differentiation, it is more efficient to produce pure cells of other layer type by differentiation.

Cell fate decision during somatic cell reprogramming has been shown to involve transitions between mesenchymal (M) and epithelial (E) states. It is unclear if similar decisions occur during the differentiation from pluripotent to somatic states.

As a basic study for the present invention, we tested this by mapping cell fate changes during the hepatic differentiation of hESCs with bulk RNA-seq and showed that hESCs start differentiation with a synchronous EMT followed by a less synchronous MET before acquiring liver-specific characteristics. We confirmed these changes at single cell resolution and further showed that the EMT is mediated by endogenously produced TGFβ through SNAI1 to initiate differentiation towards hepatocytes. Our study establishes epithelial-mesenchymal-epithelial transitions as a mechanism that drives cell fate decisions during hESC differentiation towards the haptic lineage, and further suggests that differentiation and reprogramming share similar cell fate decisions.

Reprogramming of somatic cells into pluripotent ones with defined factors not only provides a new way to generate functional cells for regenerative medicine, but also establishes a new paradigm for cell fate decisions. For the latter, a cell at a terminally differentiated state can be restored back to pluripotency under well-defined conditions fully observable through molecular and cellular tools. Indeed, the reprogramming process has been analyzed in great detail to reveal novel insights into the mechanism of cell fate changes¹⁻³. Of particular interest is the acquisition of epithelial characteristics from mesenchymal mouse embryonic fibroblasts (MEFs) commonly employed as starting cells in reprogramming experiments⁴. Termed the MET or mesenchymal to epithelial transition, we and others have described the MET as marking the earliest cellular change upon the simultaneous transduction of reprogramming factors POU5F1 (OCT4), SOX2, KLF4 and MYC into MEFs^{5,6}. However, when delivered sequentially as OK+M+S, they initiate a sequential EMT-MET process that drives reprogramming more efficiently than the simultaneous approach⁷, suggesting that the switching between mesenchymal and epithelial fates underlies the reprogramming process, i.e., the acquisition of pluripotency. We then speculated that such a sequential EMT-MET process may underlie cell fate decisions in other situations such as differentiation, generally viewed as the reversal of reprogramming with the loss of pluripotency. Here we report that a sequential EMT-MET also drives the differentiation of hESCs towards hepatocytes.

In the present invention, it is reveled that differentiation of human embryonic stem cells towards hepatocyte-like cells undergoes a sequential EMT-MET process: A TGF□ dependent mesenchymal fate transition during the differentiation of human embryonic stem cells towards DE originated cell, e.g., hepatocyte-like cells.

Hereby a method of controlling differentiation of stem cells is provided, comprising
(a) adding a TGF inhibitor or a SNAI1 inhibitor to said stem cell, thereby inhibiting said stem cell from the acquisition of endoderm character; or
(b) knocking out the SNAI1 gene of said stem cell, thereby inhibiting the said stem cell from the acquisition of endoderm character.

Said method can be used for restraining said stem cells from forming endoderm originated cells, e.g., hepatic cells, biliary ductal cells, exocrine pancreatic cells, endocrine pancreatic cells, lung cells, thyroid cells,as well as cell types of the gastrointestinal tract, during differentiation.

Said stem cells are mammalian stem cells; preferably, human embryonic stem cells or iPS cells.

Optionally, said TGF inhibitor is TGFβ inhibitor selected from the group consisting of TGFβ signaling inhibitor, TGFβ expression inhibitor, TGFβ interfering agent, TGFβ blocking antibody. As exemplified, the TGFβ inhibitor is selected from the group consisting of RepSox, SB525334.

Optionally, said TGFβ inhibitor is added with a final concentration of no less than 1 µM in the culture medium; preferably 1-20 µM, more preferably 1-10 µM. In one preferred example, it is added with a final concentration of 2 µM.

Optionally, said SNAI1 inhibitor is siRNAs to SNAI1.

In the meanwhile, a method of inhibiting the differentiation of stem cells toward endoderm originated cells is provided, comprising inhibiting the epithelial mesenchymal transition within 3 days from the beginning of differentiation, thereby inhibiting the stem cells from the acquisition of endoderm character.

On the other hand, a method of promoting the differentiation of stem cells toward endoderm originated cells is provided, comprising a stimulation of epithelial-mesenchymal transition followed by a stimulation of mesenchymal-epithelial transition.

Related therapeutic medicine can be developed using the inhibitor disclosed in the present invention.

We presented evidence here that a sequential EMT-MET process occurs during the differentiation of hESCs towards hepatic lineage, mirroring a similar mechanism for somatic cell reprogramming⁷. These findings may help provide a unified understanding of cell fate decisions in both reprogramming and differentiation. Since reprogramming is generally viewed as the reversal of differentiation, this unified mechanism appears to be quite reasonable as cells must pass through similar phases, albeit in opposite directions, hence involve similar processes and regulators. In agreement, lineage specifiers have been reported to serve as potent reprogramming factors^{13, 14}. Further studies should be directed at elucidating the underlying mechanisms that can orchestrate these cellular and molecular processes during both reprogramming and differentiation in terms of cell fate directions. On the other hand, the transitions between mesenchymal and epithelial states, i.e., EMT and MET, have been observed both in vivo and in vitro¹⁵⁻¹⁷. Their coupled role in mediating cell fate decision thus warrant further analysis at multiple levels both in vivo and in vitro and may provide insights into not only normal development, but also disease processes as well.

### BRIFE DESCRIPTION OF THE DRAWINGS

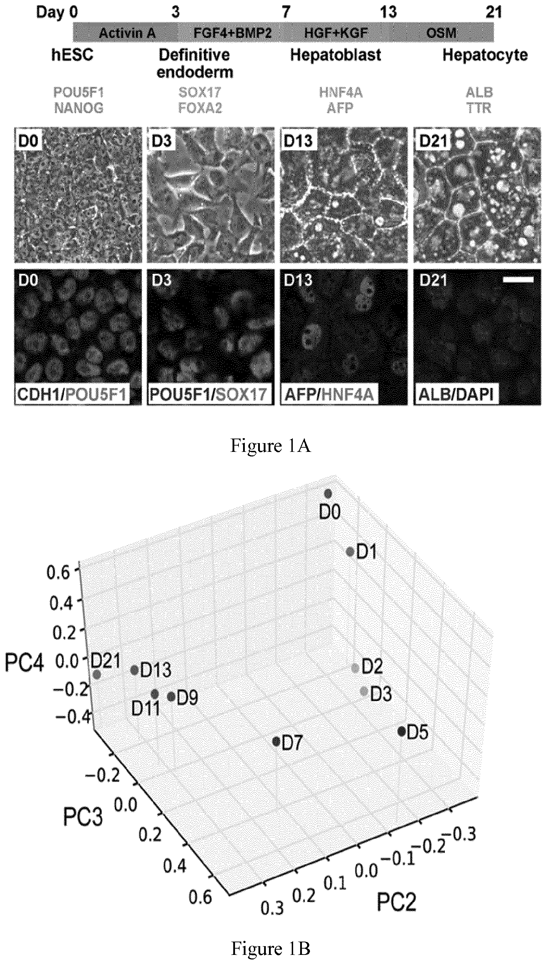

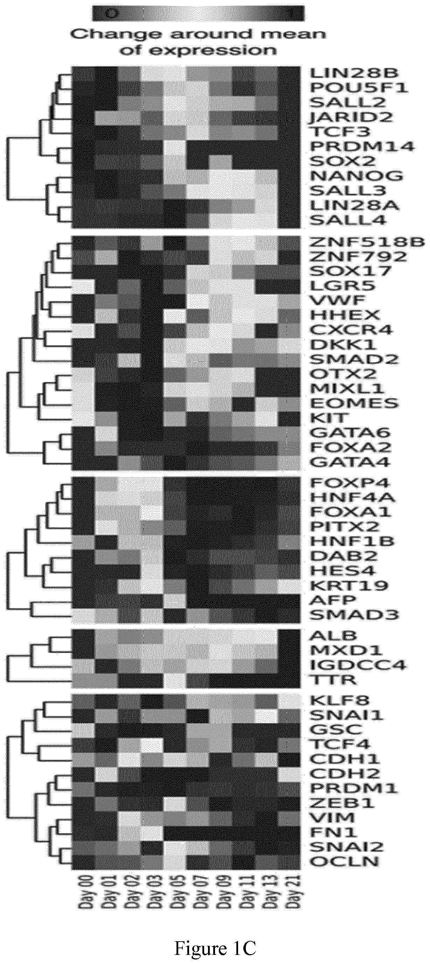

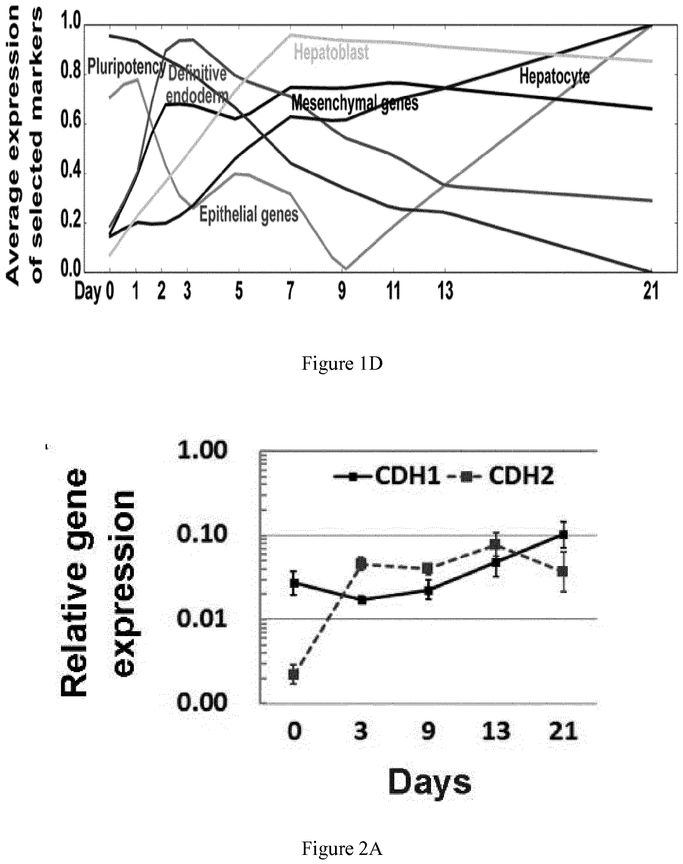

**Figure 1****. A sequential EMT-MET connects the differentiation of hESCs to hepatocytes**
   (A) Schematic of the differentiation protocol and the stages of cell differentiation. Representative live photos as well as images of immunofluorescence staining of marker molecules at the indicated stages were shown. Scale bar is 20 µm.
   (B) Principal component analysis indicates the transitions that occur during differentiation. PC2 roughly corresponds to hESC to liver acquisition and PC3 corresponds to an epithelial/mesenchymal phenotype.
   (C) Expression of selected marker genes from the RNA-seq data. Gene expression is normalized across the mean of expression over the time course. From top to bottom, the marker genes are: pluripotency, DE, hepatoblast, hepatocyte and EMT/MET.
   (D) Broad waves of EMT, differentiation and acquisition, based on the marker genes defined in (C).

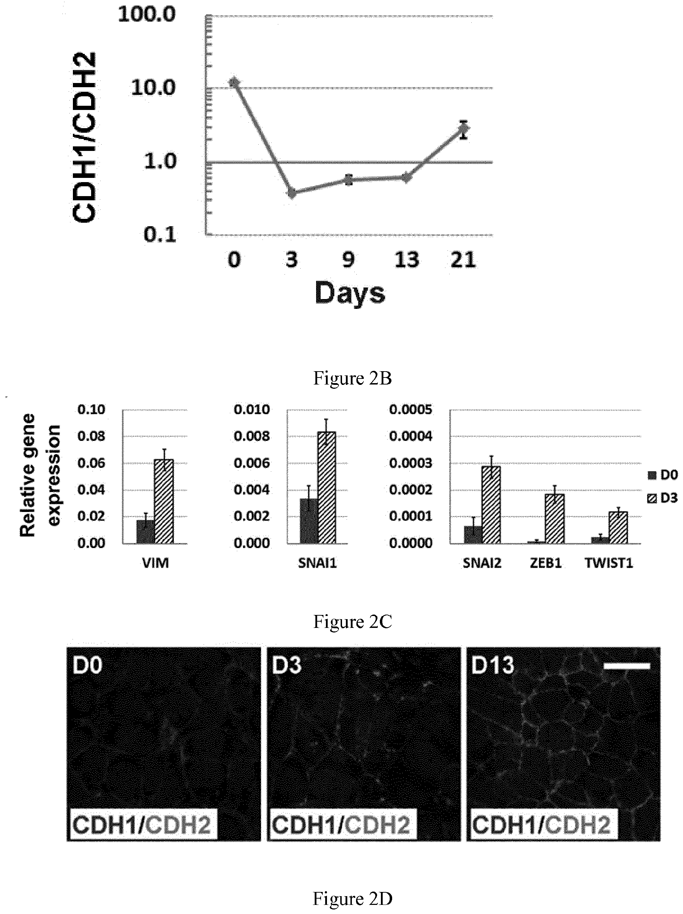

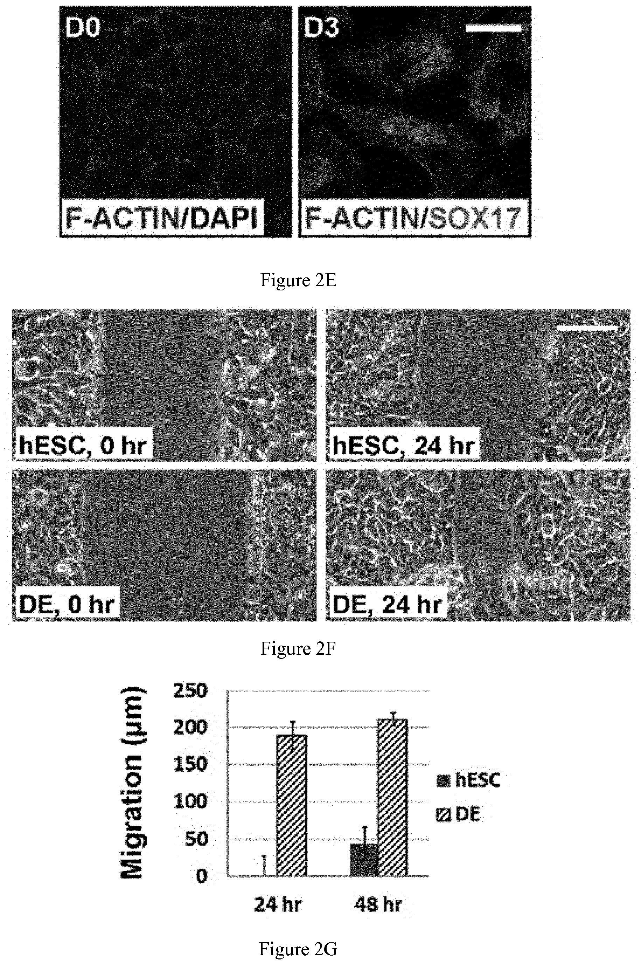

**Figure 2****. Activin A induces a transitional mesenchymal state in DE**
   (A) Dynamic expressions of CDH1 and CDH2 at the indicated stages of hepatic differentiation of hESCs.
   (B) An E-M-E conversion of cells during the hepatic differentiation. The ratio of CDH1 to CDH2 was used as an indicator of the epithelial/mesenchymal states of cells.
   (C) Relative expression levels of mesenchymal genes at D0 and D3. The expression level of GAPDH was arbitrary set as 1.
   (D) Immunofluorescence staining of CDH1 and CDH2 at the indicated differentiation stages.
   (E) Rhodamine-conjugated phalloidin staining of F-actin in day 0 and day 3 cells.
   (F-G) Migration assay for hESC and DE. Scale bars represent 20 µm in (D and E) and 100 µm in (F).

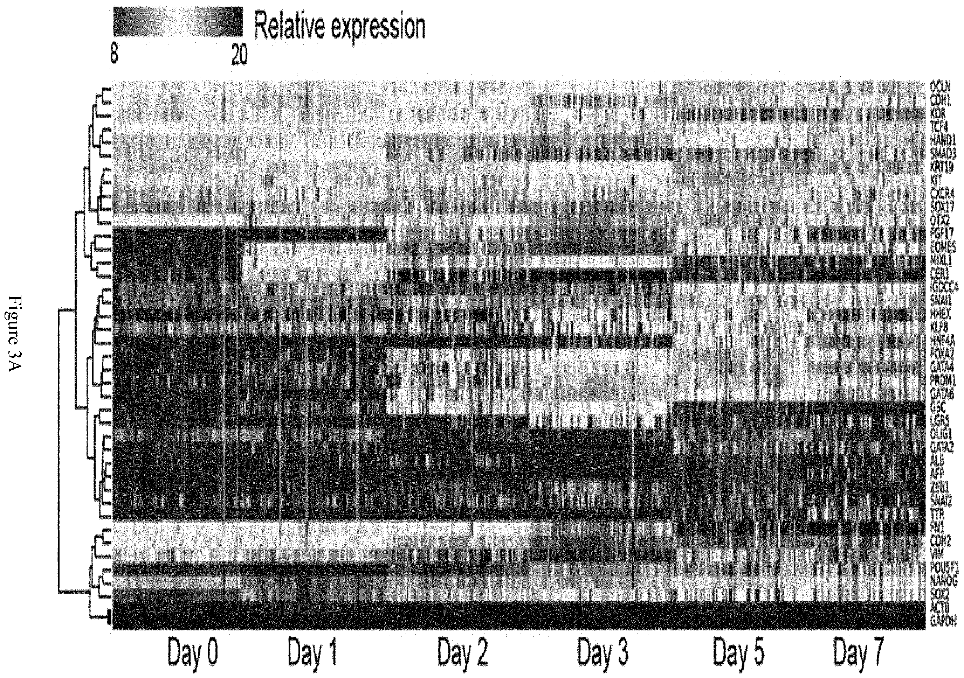

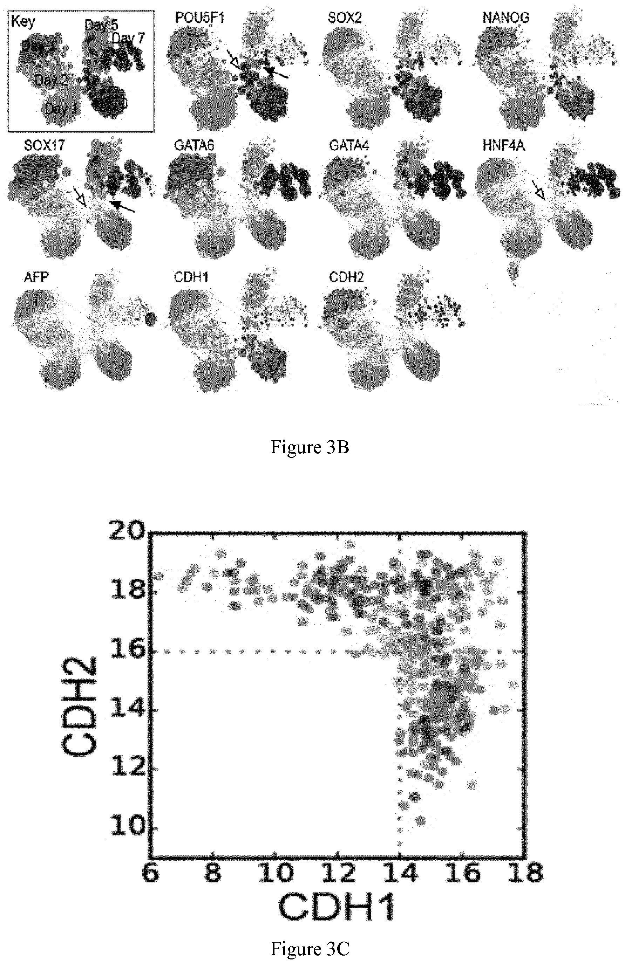

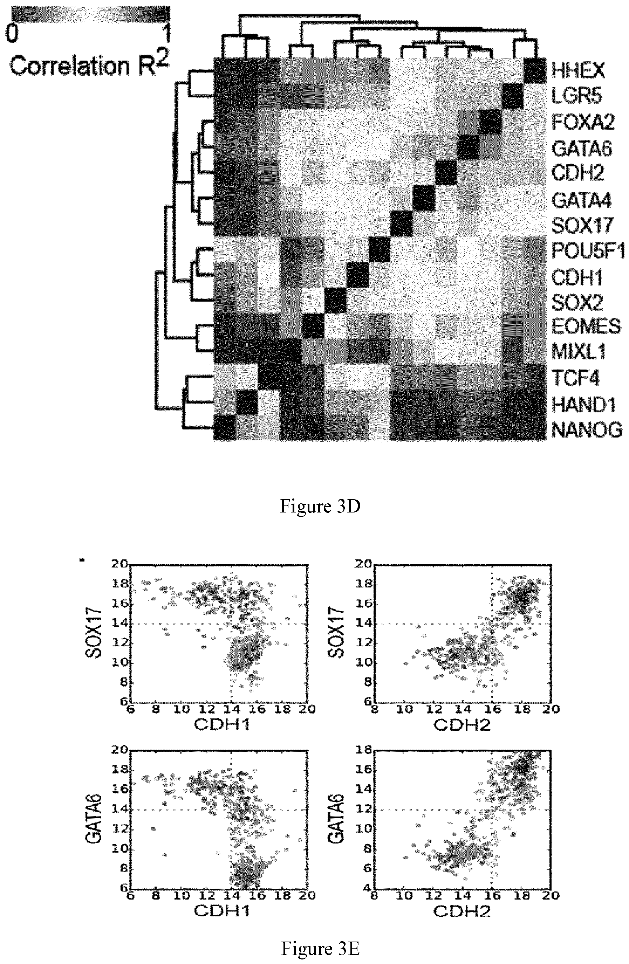

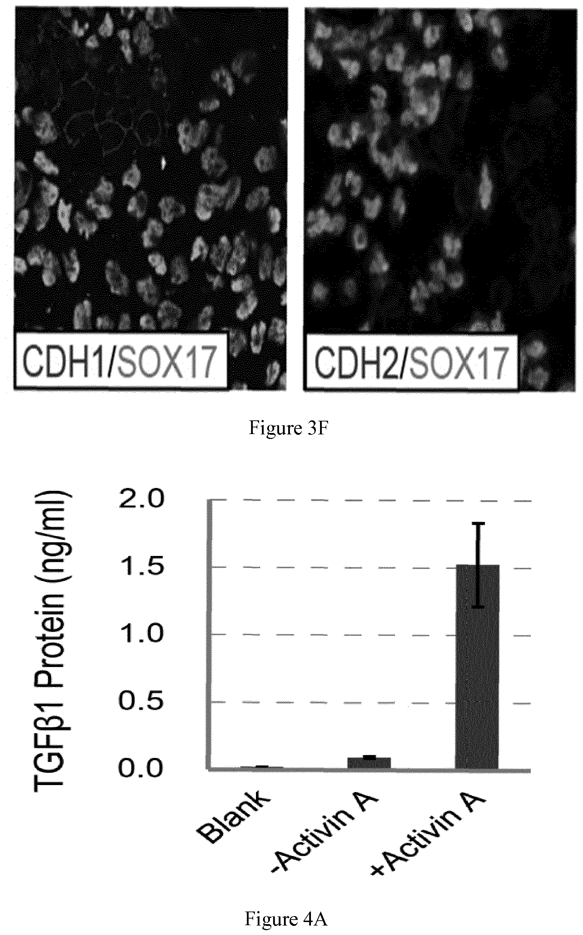

**Figure 3****. Single cell qPCR analysis reveals a synchronous EMT during the differentiation to hESCs to DE**
   (A) Heatmap of the expressions of selected genes.
   (B) Relational network plots. Different colors indicate specific days of treatment, node sizes are 2^{[relative expression]}. Open arrows indicate a population of day 5/7 hESC-like cells expressing POU5F1, SOX2, NANOG. Closed arrows indicate a population of cells simultaneously expressing the pluripotent marker genes POU5F1, SOX2, NANOG and the DE markers SOX17, GATA4 and GATA6.
   (C) Scatter plot of CDH1 versus CDH2 expression, colors are the same as in panel (B).
   (D) Correlation of gene expression for the indicated genes for days 0 through 3.
   (E) Scatter plots of single cell gene expression for SOX17 and GATA6 versus the epithelial marker gene CDH1 and the mesenchymal marker gene CDH2. Colors are the same as in panel (B).
   (F) Immunofluorescence staining of CDH1, CDH2 and SOX17 at day 3.

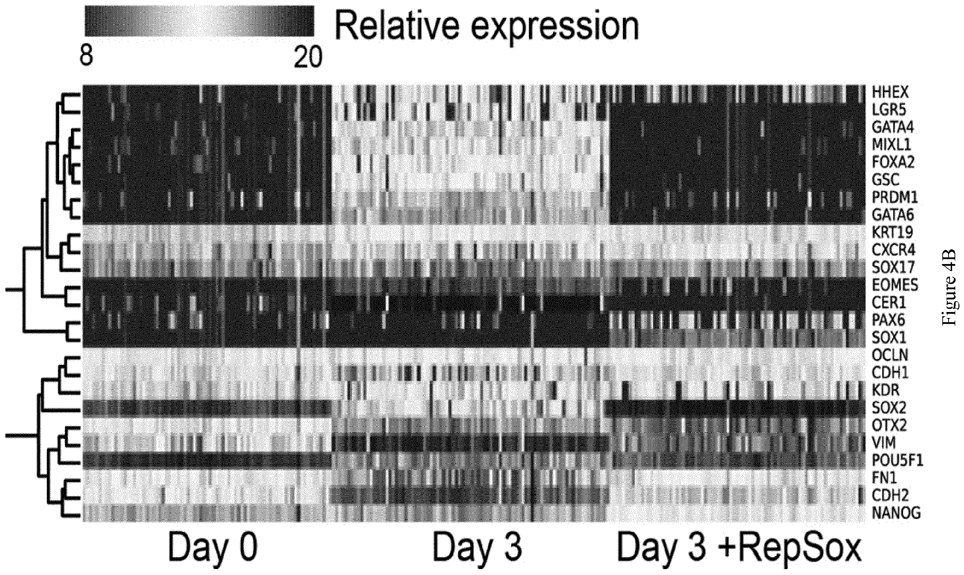

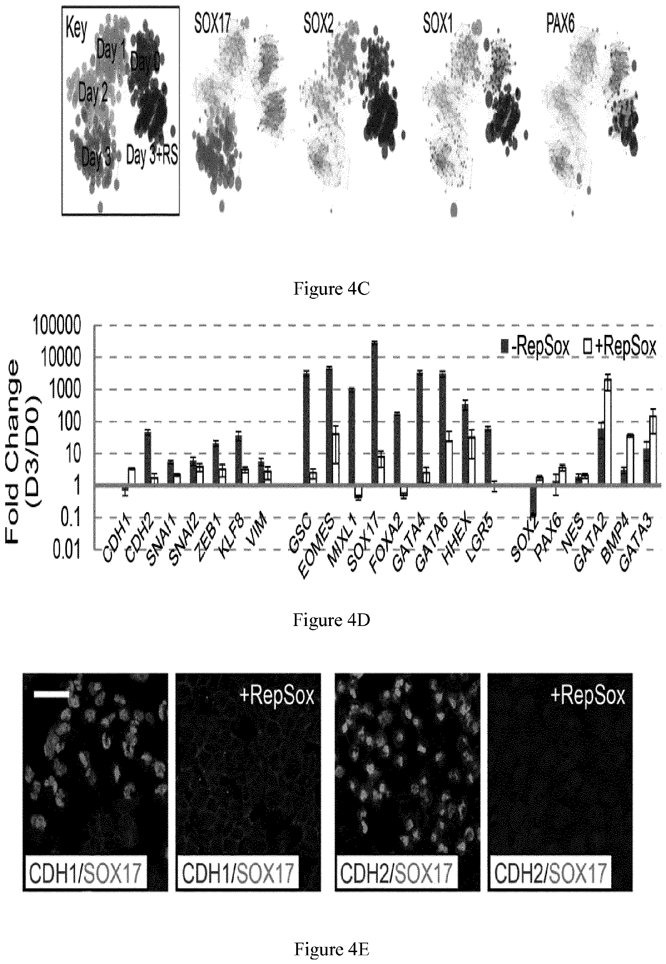

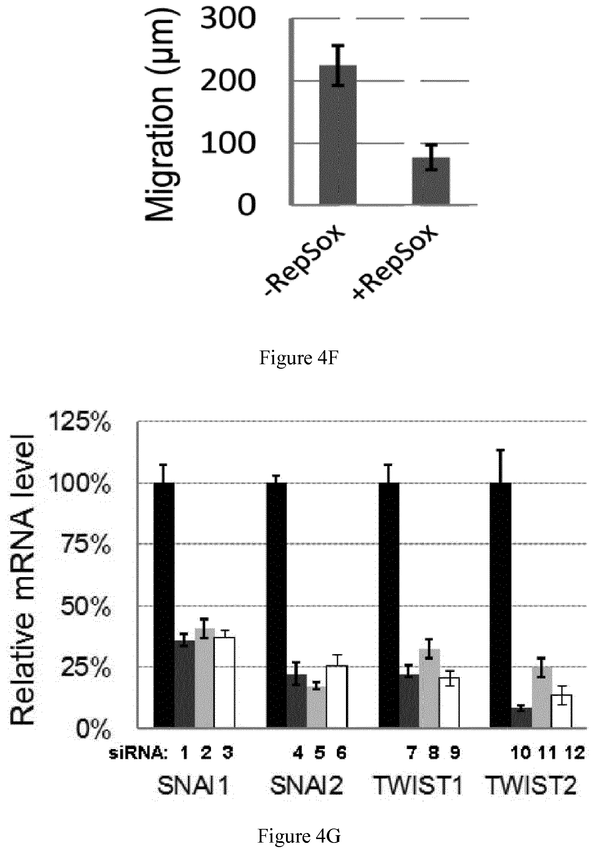

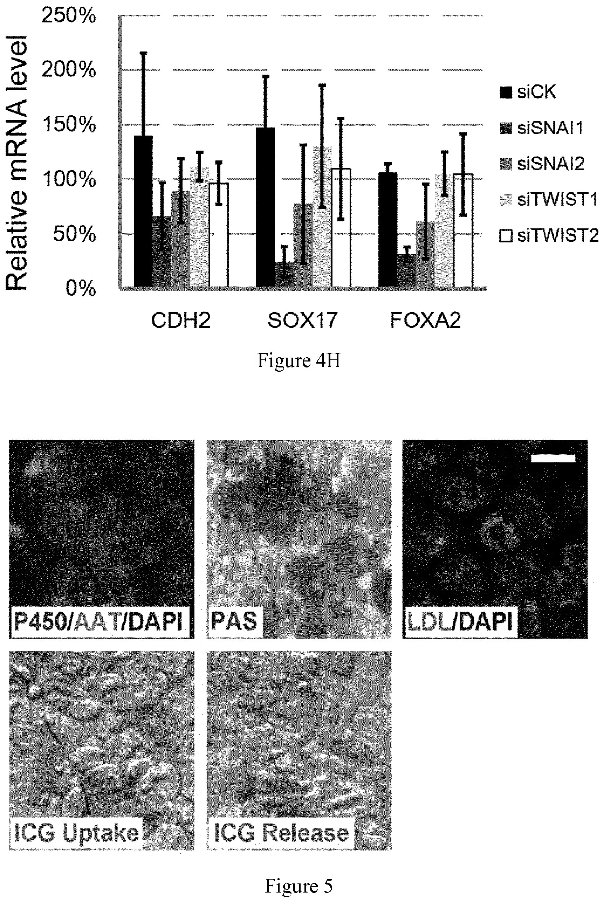

**Figure 4****. TGFβ mediates Activin A induced EMT and DE formation**
   (A) Secreted protein levels of TGFβ in culture medium.
   (B) Single-cell qPCR of selected marker genes on cells treated with RepSox.
   (C) Relational maps, constructed as in Figure 3B, node sizes are 2^{[relative expression]}.
   (D) Bulk qPCR analysis for a selection of marker genes.
   (E) Immunofluorescence staining of day 3 cells with or without RepSox. Scale bar indicates 20 µm.
   (F) Migration ability of day 3 cells. Assays were performed as in Figure 2F.
   (G) qRT-PCR analysis for the efficiencies of siRNAs to the indicated genes.
   (H) Treatment of cells with siRNAs to SNAI1 effectively blocked the induction of SOX17 and FOXA2.
**Figure 5****. Characterization of hESC derived hepato-like cells at day 21**
   (A) Immunofluorescence staining for Cytochrome P450 (Red) and Alpha-1-antitrypsin (AAT, Green).
   (B) PAS staining for glycogen.
   (C) Uptake of Alexa Fluor® 488 labeled LDL (Green).
   (D, E) Uptake and release of indocyanine green (ICG).
**Figure 6****. qRT-PCR analysis of TGFβ1 expression levels in un-induced H1 (hESCs), or in differentiation media with or without Activin A**
   The expression level of GAPDH is arbitrary set as 1. Data represent mean±SD from three independent biological repeats.
**Figure 7****. qRT-PCR analysis of the expressions of the indicated mesendoderm and endoderm markers in the control siRNA (siCK) or siRNA to SNAI1 (siSNAI1) treated cells**
   The expression level of these genes in the absence of siRNA treatment is defined as 1. Data represent mean±SD from three independent biological repeats.
**Figure 8****. A working model of EMT-MET in hepatic differentiation of hESCs**

### DETAILED DESCRIPTION

For clarity of disclosure, and not by way of limitation, the detailed description of the invention is divided into the following subsections that describe certain features, methods, embodiments or applications of the present invention.

### Experimental description

### Maintenance and hepatic differentiation of human ESCs.

Undifferentiated human H1 ES cells were maintained in monolayer culture on Matrigel (BD Biosciences, 354277) in mTeSR1 medium (Stemcell Technologies, 05850) at 37°C with 5% CO₂. Cells were manually passaged at 1:4 to 1:6 split ratios every 3 to 5 days. For hepatic differentiation, we established a serum-free protocol based on previously described protocols with minor modifications^{8,9}. Briefly, HI cells were cultured for 3 days in RPMI/B27 medium (Insulin minus, Gibco, A18956-01) supplemented with 100 ng/ml Activin A (Peprotech, A120-14E), followed by 4 days with 20 ng/ml BMP2 (Peprotech, 120-02)and 30 ng/ml FGF-4 (Peprotech, 100-31) in RPMI/B27(complete with Insulin, Gibco, 17504-044) medium, then 6 days with 20 ng/ml HGF (Peprotech, 100-39) and KGF (Peprotech, 100-19) in RPMI/B27 (complete with Insulin), then 8 days with 20 ng/ml Oncostatin-M (R&D Systems, 295-OM/CF) in Hepatocyte Culture Media (Lonza, cc-3198) supplemented with SingleQuots (without EGF).

### Immunofluorescence staining.

Cells on glass coverslips were fixed in 4% paraformaldehyde (PFA, Jingxin, GI001,Guangzhou) for 30 min, washed with PBS for 3 times and permeabilized in 0.3% Triton X-100 (Sigma, T9284) /PBS for 30 min. After two brief washes in PBS, cells were blocked in 10% FBS (ExCell Biology, FSP500) /PBS for 1 hr at RT. For actin staining, cells were incubated with 1 unit/ml rhodamine phalloidin (Invitrogen, R415) for 60 min at RT. For Immunofluorescence staining, samples were then incubated with primary antibody (diluted in 1xPBS with 0.3% Triton X-100 /10% FBS) overnight at 4°C, washed 3 times with blocking solution and incubated with a secondary antibody for 1 hr at RT. The cells were washed with blocking solution and PBS, counter stained with DAPI (Sigma, D9542) for 5 min, and then imaged with the Zeiss LSM 710 confocal microscope (Carl Zeiss). Antibodies used in this study are listed in supplementary Table S1.

### Real-time RT-PCR.

Total RNA was extracted using the Trizol reagent (MRC, TR1187) and 2 µg of total RNA was reverse-transcribed using the ReverTraAce Kit (TOYOBO, 34520B1). The product (cDNA) was properly diluted and used as PCR template. PCR reactions were performed with the SYBR®Premix Ex Taq™ Kit (TAKARA, RR420A) on the CFX96 Touch™ Real-Time PCR Detection System (Bio-Rad). GAPDH was used as the internal control. Three to four biological replicates were performed for each assay and data represents mean ± SD.

### siRNA treatment.

Three independent siRNAs for each gene were designed and synthesized by Ribobio (Guangzhou, China) to target human SNAI1, SNAI2, TWIST1, TWIST2 and ZEB1. Transfection of siRNAs was performed using the Lipofectamine RNAiMAX Reagent (Life Technologies, 13778-150) according to the manufacturer's protocol and the final concentration of each siRNA was 30 nM. Three consecutive transfections (every 48 hrs) were performed on 70∼80% confluent H1 cells maintained in mTeSR1. Cells were then treated with Activin A after the third transfection for three days as described above. The knockdown efficiency of siRNA was determined by real-time RT-PCR 96 hrs post the third transfection. Two of the most effective siRNAs to each gene were combined and used in further experiments.

### RNA-seq and bioinformatics.

Total RNA was harvested during the differentiation of hESCs to hepatocyte-like cells on days 0, 1, 2, 3, 5, 7, 9, 11, 13, and 21. Approximately 4 µg of RNA was used to generate sequencing-ready cDNA library with the TruSeq RNA Sample Prep Kit (Illumina, RS-122-2001). DNA fragments (250-300 bp) were recovered from the gel slice using the QIAquick gel extraction kit (QIAGEN, 28704). The concentration of cDNA library was determined with the Qubit® dsDNA HS Assay Kit (Invitrogen, Q32851). Samples were sequenced on a MiSeq according to the manufacturer's instructions to an average depth of 2 million sequence tags. Reads were aligned to the ENSEMBL (mm10 v76) transcriptome using Bowtie2 (v2.2.0)¹⁸ and RSEM (v1.2.17)¹⁹, GC-normalized using EDASeq (v2.0.0)²⁰. Analysis was performed using glbase²¹. Reads were deposited with GEO under the accession number: GSEXXXXXX

### Single cell qPCR and analysis.

Single cell qPCR was performed using a Fluidigm C1 and BioMark HD as described by the manufacturer. Briefly, a cell suspension of a concentration of 166∼250 K/mL was loaded into a 10-17 µm C₁™ Single-Cell Auto Prep IFC chamber (Fluidigm, PN100-5479), and cell capture was performed on the Fluidigm C₁™ System. Both the empty wells and doublet-occupied wells were excluded from further analysis. Upon capture, reverse transcription and cDNA pre-amplification were performed using the Ambion Single Cell-to-CT Kit (Life Technologies, PN 4458237) and C₁™ Single-Cell Auto Prep Module 2 Kit (Fluidigm, PN100-5519). The pre-amplified products were diluted 10-fold prior to analysis with TaqMan® Gene Expression Master Mix (Life Technologies, 4369016) and inventoried TaqMan® Gene Expression assays (20X, Applied Biosystems) in 96.96 dynamic Arrays™ on a BioMark System (Fluidigm). Inventoried TaqMan primers were used for single-cell qPCR. Relative expression was calculated as described in Buganim et al., 2012²² except that a Ct value of 25 was used for low expressed genes. Relational network plots (mdsquish) were implemented as part of glbase²¹ and will be described in detail elsewhere. Briefly, the normalized Euclidean distance between all cells was measured for singular value decomposed principal components 2, 3, 4 and 5. A network was then constructed using a threshold of 0.92 for weak links (dotted lines) and 0.99 for strong links (solid lines) with a maximum of the 50 best scoring edges per node, the network was then laid out using graphviz 'neato' layout. Node sizes are 2^{[relative expression]}.

### Cell migration assay.

Scratch assay was used to determine the migration activity of H1-derived cells. Briefly, cells in a confluent monolayer were scratched with a needle to form a cell-free zone into which cells at the edges of the wound can migrate. The denuded area was imaged to measure the boundary of the wound at pre-migration. Images of cell movement were captured at regular intervals within a 24-48 hr period for data analysis.

### ELISA.

The protein level of TGFβ in the Activin A stimulated H1 cell culture media was determined with an ELISA Kit (R&D Systems, DB100B) as described by the manufacturer.

### PAS staining.

PAS staining was performed using the PAS staining kit (Polysciences, 24200-1) according to the manufacturer's instructions.

### LDL uptake.

Cells were washed with PBS and incubated in culture medium containing 4 µg/ml low-density lipoprotein (LDL) (Invitrogen, L23380) for 30 min at 37°C. Cells were then fixed with 4% formaldehyde and stained with DAPI (Sigma, D9542). LDL uptake by cells was examined under a fluorescence microscope.

### ICG uptake and release.

Indocyanine green (ICG) (Sigma, 1340009) was dissolved in DMSO at 5 mg/ml. When cells were ready, ICG was diluted freshly in culture medium to 1 mg/ml. Diluted ICG was added to cultured cells for 30 min at 37°C. After washing with PBS, the cellular uptake of ICG was examined under a microscope. Then cells were refilled with the culture medium and incubated for 6 hrs and the release of cellular ICG was examined.

### A sequential EMT-MET connects hESCs to hepatocytes

With robust expression of E-cadherin (CDH1), hESCs should be considered as epithelial cells in a pluripotent state. Conversely, hepatocytes are also epithelial cells, but are somatic and fully differentiated. Thus, the generation of hepatocytes from hESCs should follow a process from one type of epithelial cells to another with the gradual loss of pluripotency and gain of hepatic characteristics, without the necessity to pass through a mesenchymal state. To map the cell fates along the differentiation pathway between hESCs and hepatocytes, we adopted a serum-free, chemically defined protocol of hepatic differentiation of hESCs based on the stepwise addition of Activin A, FGF4/BMP2, HGF/KGF and then Oncostatin M^{8,9}. As shown in Figure 1A, there were distinct stages marked by POU5F1/NANOG (pluripotent), SOX17/FOXA2 (definitive endoderm; DE), HNF4A/AFP (hepatoblasts) and ALB/TTR (hepatocytes) at days 0, 3, 13 and 21. To characterize the molecular signature of this procedure, we performed RNA-seq for days 0, 1, 2, 3, 5, 7, 9, 11, 13, and 21. Based on principal component analysis, we can see a clear epithelial state starting with hESCs, which then transitions into a mesenchymal state at day 3 before reverting back to an epithelial state (Figure 1B). This sequential EMT-MET was further supported with detailed molecular markers analyzed in Figure 1C. Indeed, these gene expression patterns can be used to model the relative stages of commitment during the differentiation system (Figure 1D), indicating a well-coordinated dynamic change from the epithelial pluripotent state to mesenchymal state and then to the epithelial hepatoblasts/hepatocytes. In addition to the EMT-MET, we also observed robust and dynamic changes of genes specific to the pluripotent, DE, hepatoblast and hepatocyte fates (Figure 1D). These results establish that a sequential epithelial-mesenchymal-epithelial transition underlies the differentiation of hESCs to hepatocytes, mirroring a similar EMT-MET process uncovered for reprogramming⁷.

### A transitional mesenchymal state during differentiation

The mesenchymal state uncovered by bulk RNA-seq in Figure 1 is defined by the robust expression of mesenchymal marker genes such as CDH2 (N-cadherin) and SNAI1 and the rapid loss of the epithelial CDH1 (E-cadherin) and down-regulation of pluripotent marker genes, such as POU5F1 and NANOG. While similar changes were also noticed during the Activin A induced in vitro differentiation of hESCs to DE^{10, 11}, it is not clear if these transitional cells are completely committed to a mesenchymal phenotype or have simply down-regulated CDH1 expression without the acquisition of genuine mesenchymal and migratory function. To further characterize this mesenchymal state, we plotted CDH1 and CDH2 expression during differentiation and showed that day 3 marks CDH1 down- and CDH2 up-regulation (Figure 2A), i.e., the down-regulation of epithelial characteristics and the concomitant acquisition of mesenchymal ones. At the protein level, we stained for CDH1 and CDH2 to show almost complete absence of CDH1 at day 3 and the gain of CDH2 in the meantime (Figure 2D). Interestingly, the cells at later days become simultaneously positive for both CDH1 and CDH2, suggesting that the expression of CDH1 and CDH2 are not mutually exclusive (Figure 2D). We then compared the expression levels of a panel of mesenchymal genes such as VIM, SNAI1, SNAI2, ZEB1 and TWIST 1 between day 0 and 3 and revealed the up-regulation of these genes in contrast to the down-regulation of CDH1 (Figure 2C). Phalloidin staining of F-actin in day 3 cells showed a pattern of actin filaments typical of mesenchymal cells (Figure 2E). To further prove these cells are indeed mesenchymal, we performed a scratch assay that indicates that only the day 3 cells are capable of migration, compared to the complete absence of motility for day 0 hESCs (Figure 2F and 2G). Taken together, we conclude that the cells at day 3 are indeed mesenchymal.

### hESCs begin differentiation with a near synchronous EMT

The Bulk RNA-seq dataset presented in Figure 1B reveals a global epithelial to mesenchymal and then to an epithelial fate change. While we can tease apart the distinct patterns from pluripotent to hepatic states through E-M-E phases in a time-dependent fashion (Figure 1C), the bulk approach can not reveal heterogeneity in the differentiation process. To resolve this process further at the single cell resolution, we performed single-cell qPCR with 46 selected genes and 2 control genes (GAPDH, ACTB) on 501 cells (Figure 3A) and constructed relational networks of the gene expression of all cells (Figure 3B). POU5F1 is initially up-regulated upon the entry to endoderm but is then down-regulated by day 3, although both POU5F1 and NANOG are not extinguished and low levels persist through to day 7 of the differentiation (Figure 3B) in agreement with the bulk RNA-seq (Figure 1C). As expected, SOX17 and GATA6 are up-regulated by day 3 and GATA4 and HNF4A are induced slightly later (Figure 3B). Day 3 cells show substantial homogeneity in their response to Activin A and acquisition of DE-character (R²=0.74 compared with the bulk RNA-seq), suggesting a near synchronous traversal through the EMT. This is remarkable, perhaps reflecting either homogeneous starting hESCs or the synchronization power of Activin A.

Surprisingly, the synchrony appears to break down at day 5 with many cells simultaneously expressing CDH1 and CDH2 and the correlation with the bulk is also considerably reduced (R²=0.45), suggesting substantial heterogeneity. In agreement with the synchronous to asynchronous transition, the starting EMT is exclusive: cells at day 3 express only CDH2, but the following MET is heterogeneous and many cells express both CDH1 and CDH2 at day 5 and only a small number of cells at day 7 no longer express CDH2 (Figure 3B and 3C).

Interestingly, at day 5 and day 7, we can find small numbers (11 and 12, respectively) of hESC-like cells. These cells uniformly express POU5F1, SOX2, NANOG and CDH1 and do not express HNF4A or SOX17 (Figure 3B, open arrow), suggesting that they are perhaps resistant to differentiation or the differentiated hESCs retain some epigenetic memory of their previous state and somehow revert to a state more similar to undifferentiated hESCs. In addition, at day 5 and 7 there are 11 and 5 cells respectively that simultaneously express POU5F1, SOX2, NANOG, SOX17, GATA4 and GATA6 (Figure 3B, closed arrow), indicating incomplete commitment. Although some cells at day 5 start to express the hepatoblast marker gene HNF4A, it is widely expressed at day 7. But, day 7 cells show substantial heterogeneity (R²=0.29 against bulk RNA-seq), with a mixture of hESC-like cells (n=12), mixed endoderm precursor cells (n=5), hepatoblast cells (n=62) and even a few definitive hepatocyte-like cells expressing low levels of AFP (n=5) (Figure 3B). Nevertheless, these single cell analyses also revealed a sequential EMT-MET process between hESCs and hepatocyte-like cells, in agreement with bulk-seq dataset.

### EMT precedes the acquisition of definitive endoderm

We then wished to resolve the relationship between EMT and the acquisition of DE markers. A previous report indicated that the EMT could be uncoupled from DE markers when EOMES was knocked-down¹¹. Here we took advantage of the single cell-qPCR analysis, which when organized into relational maps suggests the temporal order of DE acquisition and EMT. When we clustered the single-cell qPCR correlated gene expression for days 0-3 (Figure 3D), we detected two major clusters centered on either CDH1 or CDH2. The CDH1 cluster contained POU5F1 and SOX2, i.e., the pluripotent state, while the CDH2 cluster contained the major DE marker genes FOXA2, GATA6, GATA4 and SOX17. To map the precise timing in more detail we plotted scatter plots of individual cell expression of CDH1 or CDH2 against the DE marker genes SOX17 and GATA6 (Figure 3E). We found that SOX17 and GATA6 expression is mutually exclusive with CDH1 expression at day 3, whilst conversely SOX17 and GATA6 are both coincident with CDH2. To further resolve the transitions, we then performed immunofluorescence analysis and showed that SOX17 positive cells were all negative for CDH1, but were positive for CDH2 (Figure 3F). More importantly, there were CDH2-positive but SOX17-negative cells, suggesting that EMT has occurred but SOX17 has not yet been sufficiently induced in these cells (Figure 3F). These results indicated that EMT precedes the specification of DE.

### Endogenous TGFβ activated by Activin A drives EMT and DE formation

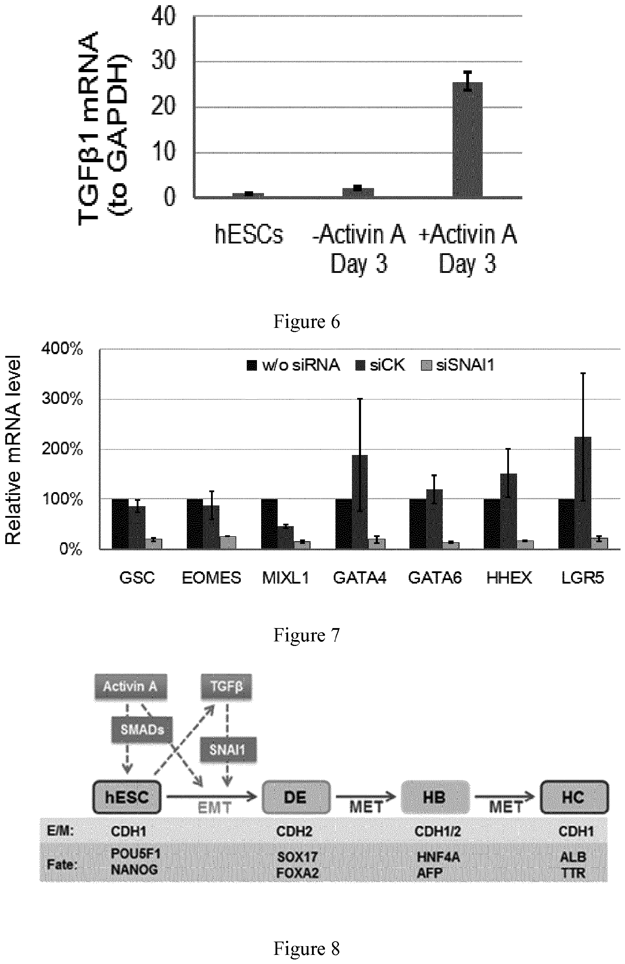

We were intrigued by the rapid and synchronous EMT at the start of the differentiation process. However, Activin A is not a strong inducer of EMT so it might function through the stimulation of other EMT-inducing signals. We previously reported that TGFβ is a major barrier for reprogramming MEFs into iPSCs: It must be suppressed by reprogramming factors for the MET and somatic reprogramming to occur⁵. Our RNA-seq data indicated that TGFβ was strongly induced by Activin A treatment so the endogenously produced TGFβ might induce EMT during the formation of DE. To test this hypothesis, we measured TGFB1 gene expression by qRT-PCR and showed a robust activation of TGFB1 by Activin A at day 3 (Figure 6). Consistently, we can detect physiological levels of TGFβ protein (1.5 ng/ml) in the culture media of day 3 cells by ELISA assay (Figure 4A). To investigate its role in EMT, we added the TGFβ-signaling inhibitor RepSox¹² to cells and showed by single cell-qPCR analysis that it not only blocked the EMT but also the formation of DE (Figure 4C; Table S5). Remarkably, day 3 Activin A cells treated with RepSox stayed very close to hESCs and continued to express POU5F1 and NANOG (Figure 4C), indicating that a TGFβ mediated EMT is required for the exit of the pluripotent state for Activin A treated hESCs. In fact, these cells maintained the expression of CDH1 and SOX2 at the same level as hESCs (Figure 4C). While Activin A-treated cells at day 3 exhibited robust SOX17 expression, RepSox blocked its induction completely (Figure 4C). On the other hand, ectodermal markers such as SOX1 and PAX6 were slightly induced in the RepSox treated cells (Figure 4C). These results indicate that Activin A treated hESCs in the presence of RepSox failed to undergo EMT and differentiate into DE.

We further confirmed the role of TGFβ by analyzing EMT and other lineage markers by qRT-PCR and showed that RepSox blocked the expression of CDH2, SNAI1, SNAI2, ZEB1, KLF8 and VIM while maintaining the expression of CDH1, blocked the expression of mesendoderm/endoderm markers such as GSC, EOMES, MIXL1, SOX17, FOXA2, GATA4, GATA6, HHEX and LGR5 (Figure 4D). Of note, other lineage markers such as PAX6, GATA2, BMP4 and GATA3 appeared to be mildly up-regulated, suggesting an alternate mesoderm/neuroectoderm cell fate. The mutual exclusivity between CDH1 and SOX17, and the co-regulation between CDH2 and SOX17 with or without RepSox were further confirmed by immunostaining (Figure 4E). We then measured the migration ability of these cells and found that day 3 cells induced by Activin A in the presence of RepSox were less motile when compared to DE cells (Figure 4H). These results demonstrate that TGFβ is required not only for the EMT, but also the formation of DE in Activin A induced hESCs.

### SNAI1 is required for EMT and DE formation

We then turned out attention to transcriptional factors up-regulated at day 3 and decided to focus on SNAI1 and TWIST family members based on RNA-seq dataset (Figure 1C and 2C). We first designed siRNAs to these genes and tested their knockdown efficiency by qRT-PCR (Figure 4G). We then transfected cells with the combination of two of the most effective siRNAs for each gene and determined the effect of target gene knockdown on hESC differentiation. We showed that, among the candidate genes, SNAI1 was most critical for the activation of CDH2 as well as DE markers such as SOX17 and FOXA2 (Figure 4H). In addition, the expression of all mesendoderm/endoderm makers examined were similarly suppressed when SNAI1 was knocked-down (Figure 7). These results indicated that SNAI1 is indispensible for EMT and the specification of DE cell fate. Together, our data suggests that Activin A induces an autocrine production of TGFβ, which in turn induces a SNAI1-mediated EMT program, including an obligatory mesenchymal phase during the hepatic differentiation ofhESCs.

### EXAMPLES

The following examples are provided for the purpose of illustrating the invention, and should not be construed as limiting.

### Example 1 Inhibition of differentiation of hESCs towards definitive endoderm using TGFβ inhibitor.

Culturing condition during differentiation is as follows. Nearly 50% confluent undifferentiated human HI ES cells obtained from ATCC were passaged by Accutase 24hr after seeding, cells were almostly 90% confluent at 37°C with 5% CO₂. Then HI cells were cultured for 3 days in RPMI/B27 medium (Insulin minus) supplemented with 100 ng/ml Activin A in the presence of 2 µM Repsox or not.

TGFβ-signaling inhibitor RepSox was added with a final concentration of 2 µM at day 0 from the start of differentiation of hESCs.

Expression of various markers of definite layer type cells, i.e., SOX1 / PAX6 (ectoderm markers), GATA2/BMP4 (mesoderm markers), and SOX17/FOXA2 (endoderm markers) were tested during differentiation to evaluate the differentiated cells.

Techniques such as single cell qPCR and bioinfomatics, immunofluorescence staining and real-time RT-PCR are used to evaluate the effect of TGFβ inhibitor on hepatic differentiation of hESCs. A detailed description of the methods is presented in the experimental description section.

The results are presented in Figure 4B-E. POU5F1 / NANOG which is marker of hESCs, SOX1 / PAX6 which is marker of ectodermal, and GATA2 / GATA3 / BMP4 which is marker of mesoderm, was expressed (Figure 4B-D). While in the meantime, no endoderm marker is expressed.

As control group, differentiation of hESCs was conducted using the same culturing conditions but without the addition of TGFβ-signaling inhibitor. Most of the hESCs-derived cells robustly expressed endodermal markers, such as SOX17, FOXA2, GATA4, and GATA6, while the markers of other layer type cells were hardly detected (Figure 4B-D).

It can be concluded that the addition of TGFβ-signaling inhibitor can inhibit the differentiation of hESCs from the formation of definitive endoderm originated cells.

In the meanwhile, TGFβ inhibitor enhances the expression of ectoderm and mesoderm markers (Figure 4B-D).

### Example 2 Inhibition of differentiation of hESCs towards definitive endoderm using siRNAs to SNAI1.

Culturing condition during differentiation is as follows. H1 cells after the third transfection were cultured for 3 days in RPMI/B27 medium (Insulin minus) supplemented with 20 ng/ml Activin A.

Expression of various markers of definitive layer type cells, i.e., SOX17, FOXA2, GSC, GATA4, and GATA6, were tested during differentiation to evaluate the differentiated cells.

Methods include siRNA treatment and real-time RT-PCR. A detailed description of the methods is presented in the experimental description section.

The results are presented in Figure 4G-H and Figure 7. Among the candidate genes, SNAI1 was most critical for the activation of CDH2 as well as DE markers such as SOX17 and FOXA2 (Figure 4H). In addition, the expression of all mesendoderm/endoderm makers examined were similarly suppressed when SNAI1 was knocked-down (Figure 7). These results indicated that SNAI1 is indispensible for EMT and the specification of DE cell fate. The inhibition or knockdown of SNAI1 can inhibit the differentiation of hESCs from the formation of definitive endoderm originated cells.

### References and Prior Art

1. Polo, J.M. et al. A molecular roadmap of reprogramming somatic cells into iPS cells. Cell 151, 1617-1632 (2012).
2. Hussein, S.M. et al. Genome-wide characterization of the routes to pluripotency. Nature 516, 198-206 (2014).
3. Hansson, J. et al. Highly coordinated proteome dynamics during reprogramming of somatic cells to pluripotency. Cell reports 2, 1579-1592 (2012).
4. Shu, X. & Pei, D. The function and regulation of mesenchymal-to-epithelial transition in somatic cell reprogramming. Current opinion in genetics & development 28, 32-37 (2014).
5. Li, R. et al. A mesenchymal-to-epithelial transition initiates and is required for the nuclear reprogramming of mouse fibroblasts. Cell stem cell 7, 51-63 (2010).
6. Samavarchi-Tehrani, P. et al. Functional genomics reveals a BMP-driven mesenchymal-to-epithelial transition in the initiation of somatic cell reprogramming. Cell stem cell 7, 64-77 (2010).
7. Liu, X. et al. Sequential introduction of reprogramming factors reveals a time-sensitive requirement for individual factors and a sequential EMT-MET mechanism for optimal reprogramming. Nat Cell Biol 15, 829-838 (2013).
8. Song, Z. et al. Efficient generation of hepatocyte-like cells from human induced pluripotent stem cells. Cell research 19, 1233-1242 (2009).
9. Si-Tayeb, K. et al. Highly efficient generation of human hepatocyte-like cells from induced pluripotent stem cells. Hepatology 51, 297-305 (2010).
10. D'Amour, K.A. et al. Efficient differentiation of human embryonic stem cells to definitive endoderm. Nature biotechnology 23, 1534-1541 (2005).
11. Teo, A.K. et al. Pluripotency factors regulate definitive endoderm specification through eomesodermin. Genes & development 25, 238-250 (2011).
12. Ichida, J.K. et al. A small-molecule inhibitor of tgf-Beta signaling replaces sox2 in reprogramming by inducing nanog. Cell stem cell 5, 491-503 (2009).
13. Shu, J. et al. Induction of pluripotency in mouse somatic cells with lineage specifiers. Cell 153, 963-975 (2013).
14. Montserrat, N. et al. Reprogramming of human fibroblasts to pluripotency with lineage specifiers. Cell stem cell 13, 341-350 (2013).
15. Nieto, M.A. Epithelial plasticity: a common theme in embryonic and cancer cells. Science 342, 1234850 (2013).
16. Thiery, J.P., Acloque, H., Huang, R.Y. & Nieto, M.A. Epithelial-mesenchymal transitions in development and disease. Cell 139, 871-890 (2009).
17. Polyak, K. & Weinberg, R.A. Transitions between epithelial and mesenchymal states: acquisition of malignant and stem cell traits. Nat Rev Cancer 9, 265-273 (2009).
18. Langmead, B. & Salzberg, S.L. Fast gapped-read alignment with Bowtie 2. Nature methods 9, 357-359 (2012).
19. Li, B. & Dewey, C.N. RSEM: accurate transcript quantification from RNA-Seq data with or without a reference genome. BMC bioinformatics 12, 323 (2011).
20. Risso, D., Schwartz, K., Sherlock, G. & Dudoit, S. GC-content normalization for RNA-Seq data. BMC bioinformatics 12, 480 (2011).
21. Hutchins, A.P., Jauch, R., Dyla, M. & Miranda-Saavedra, D. glbase: a framework for combining, analyzing and displaying heterogeneous genomic and high-throughput sequencing data. Cell regeneration 3, 1 (2014).
22. Buganim, Y. et al. Single-cell expression analyses during cellular reprogramming reveal an early stochastic and a late hierarchic phase. Cell 150, 1209-1222 (2012).

## Claims

1. A method of controlling differentiation of stem cells comprising
(c) adding a TGF inhibitor or a SNAI1 inhibitor to said stem cell, thereby inhibiting said stem cell from the acquisition of endoderm character; or
(d) knocking out SNAI1 gene of said stem cell, thereby inhibiting said stem cell from the acquisition of endoderm character.

2. The method of claim 1, wherein said method is used for restraining said stem cells from forming endoderm originated cells during differentiation.

3. The method of claim 2, wherein said endoderm originated cells are hepatic cells, biliary ductal cells, exocrine pancreatic cells, endocrine pancreatic cells, lung cells, thymocyte, thyroid cells, and/or cells of gastrointestinal tract.

4. The method of claim 1, wherein said stem cells are mammalian stem cells; preferably, human embryonic stem cells or iPS cells.

5. The method of any of the claims above, wherein said TGF inhibitor is selected from the group consisting of TGFβ signaling inhibitor, TGFβ expression inhibitor, TGFβ interfering agent, and TGFβ blocking antibody; preferably said TGFβ inhibitor is selected from the group consisting of RepSox and SB525334; more preferably said inhibitor is added with a final concentration of no less than 1 µM; or preferably said SNAI1 inhibitor is siRNAs to SNAI1.

6. A formulation for inhibiting the differentiation of stem cells toward endoderm originated cells, or a formulation for promoting the differentiation of stem cells toward mesodermal originated cells or ectoderm originated cells, wherein said formulation is TGFβ inhibitor or SNAI1 inhibitor.

7. Use of TGFβ inhibitor or SNAI1 inhibitor in the preparation of formulation for inhibiting the differentiation of stem cells toward endoderm originated cells, or formulation for promoting the differentiation of stem cells toward mesodermal originated cells or ectoderm originated cells.

8. A method of inhibiting the differentiation of stem cells toward endoderm originated cells, comprising inhibiting the epithelial-mesenchymal transition within 3 days from the beginning of differentiation, thereby inhibiting the stem cells from the acquisition of endoderm character.

9. A method of promoting the differentiation of stem cells toward endoderm originated cells, comprising a stimulation of epithelial-mesenchymal transition followed by a stimulation of mesenchymal-epithelial transition; or, comprising a stimulation of expression of TGFβ and/or SNAI1.

10. A method of any of claim 8-9, wherein said endoderm originated cells are hepatic cells, biliary ductal cells, exocrine pancreatic cells, endocrine pancreatic cells, lung cells, thymocyte, thyroid cells, and/or cells of gastrointestinal tract.
